# EUROPEAN PATENT SPECIFICATION

(11) **EP 1 827 573 B1**
(45) Date of publication and mention of the grant of the patent: **24.04.2013**
(21) Application number: 04800264.6
(22) Date of filing: 02.11.2004
(51) Int. Cl.: A61N 1/05, A61N 1/08, A61N 1/36, A61N 1/37, A61N 1/372, A61B 5/06

(54) **DEVICE FOR EVALUATING POSITIONS OF AN IMPLANTABLE MEDICAL DEVICE**
VORRICHTUNG ZUR BEURTEILUNG DER POSITIONEN EINES IMPLANTIERBAREN MEDIZINPRODUKTS
DISPOSITIF PERMETTANT D'EVALUER LES POSITIONS D'UN DISPOSITIF MEDICAL IMPLANTABLE

(43) Date of publication of application: 05.09.2007
(73) Proprietor: St. Jude Medical AB, 175 84 Järfälla (SE)
(72) Inventor: OBEL, Martin, S-182 53 Danderyd (SE)
(74) Representative: Sjölander, Henrik
(86) International application number: PCT/SE2004/001601
(87) International publication number: WO 2006/049539

(56) References cited:
- WO-A1-98/26715
- US-A- 6 161 039
- US-A1- 2002 045 809
- US-A1- 2002 169 484
- US-B1- 6 496 715

## Description

### TECHNICAL FIELD

The present invention generally relates to implantable medical devices such as implantable cardiac pacemakers and in particular to a device and a method for obtaining information related to the heart pumping activity at different lead positions within a heart and for using the information to evaluate the different lead positions in order to identify the optimal position with respect to the heart pumping activity.

### BACKGROUND OF THE INVENTION

The technology of cardiac pacemakers has developed in sophistication and functionality over the years. In general, cardiac pacemakers are designed to control the heart by correcting or compensating for various heart abnormalities which can be encountered in human patients. For example, cardiac pacemakers may provide therapeutic stimulation to the heart by delivering therapeutic pulses such as pacing, cardioversion or defibrillation pulses.

Commonly, the pulses are delivered to the heart via electrodes disposed on implantable leads coupled to the pacemaker. The pacemaker performs various sensing and pulsing functions by receiving and delivering signals through the leads. The placements of the electrodes, i.e. the leads, with respect to one or more cardiac locations is such so that the desired electrical functions such as stimulation or heart signal sensing are ensured. For example, the leads may position the electrodes with respect to one or more cardiac locations so that the pacemaker can deliver pulses to the appropriate locations of the heart.

Leads may be placed in one or more of a variety of different cardiac locations. In particular, the placement of the leads may be dependent on the cardiac conditions of the patient and the therapy to be delivered.

A proper placement of the leads, i.e. the electrodes, is essential because both the desired electrical functions, i.e. stimulation or heart signal sensing, and the desired heart muscle reaction (activity propagation) are dependent on the position of the lead, However, it is often difficult to determine whether a lead has been properly positioned and adequate tissue contact has been achieved. Today, pacemaker leads are normally checked during the implantation to ensure satisfactory electrical performance by use of a PSA (pacemaker system analyzer). A PSA is an external equipment connected to the implanted leads. In general three electrode and lead properties are checked by use of a PSA to be satisfactory;
1. Stimulation threshold. That the required electrical stimulation energy to activate the heart is low enough.
2. Heart signal. That the spontaneous heart signal picked up by the lead system is of enough amplitude.
3. Lead impedance. That the conductive path of the lead(s) together with body fluids and tissue is in physical good condition.

Furthermore, it is often difficult to get the lead to the proper or desired site, specially the left ventricular lead through the coronary sinus, partly due to technical difficulties and anatomic differences, but also due to the lack of a standard procedure for identifying the optimal or best stimulation spot for that specific patient. One method for left ventricular lead placement includes so called venogram, where a fluoro-visible dye is injected into the veins so that the veins are visible using a fluoroscopic device.

Thus, there is a need of an improved device and method that, in an efficient way, is capable of identifying the optimal position of the lead or the leads within a heart with respect to the heart pumping activity during an implantation procedure of an implantable medical device.

US 2002/0169484 discloses an apparatus for treatment of congestive heart failure. A multi-electrode having three or more selective electrodes is used together with the apparatus for identifying an optimal subset of electrodes. A three dimensional map of electrode placement may be calculated and a subset of the available electrodes in the right side of the heart is selected for simulation.

### BRIEF DESCRIPTION OF THE INVENTION

Thus, an object of the present invention is to provide a simplified and improved device for finding the optimal lead placement for one or more leads with respect to heart pumping activity during an implantation procedure. After the lead or leads have been positioned at an optimal site they are disconnected from the device and connected to the permanent pulsegenerator to be implanted. The device may be an improved PSA or a separate device.

This and other objects are achieved according to the present invention by providing a device having the features defined in the independent claim. Preferable embodiments of the invention are characterised by the dependent claims.

Thus, the invention is based on the idea of utilizing signals characteristic of the heart pumping activity to evaluate different positions of a medical lead or medical leads during an implantation procedure in order to identify the optimal site or placement for the lead or the leads with respect to a desired heart muscle reaction. That is, physiologic parameters reflecting hemodynamic performance are derived and evaluated for each lead position in order to determine the optimal site.

This solution provides several advantages over the existing solutions. One advantage is that the optimal site for placing the lead (or the leads) can be accurately determined with respect to anatomic differences of a specific patient, the specific therapy to be delivered and the desired heart muscle reaction (activity propagation).

Another advantage is that the lead site optimization is incorporated into the implant procedure. This provides for significant time savings in comparison with prior art solutions, which, in turn, reduces the risk for infection because the time required for the implantation procedure is decreased.

In a preferred embodiment of the present invention, the the bipolar right ventricular impedance is measured in order to detect the contraction of the right ventricle. At the same time the left ventricular impedance is measured in order to detect the contraction of the left ventricle. The left ventricular impedance may be measured between a left ventricular electrode placed in a coronary vein and a right ventricular electrode. The impedance changes in this configuration is mainly due to the variable left ventricular volume during the heart cycle. The lead position or positions resulting in the best synchrony between the right ventricular contraction and the left ventricular contraction is considered as the best lead position. It should however be observed that the V-V interval should be optimized before any tests are performed to evaluate the merits of the actual lead position is evaluated. For biventricular pacing the left ventricular lead position is generally the most important lead position. In a typical case the RV lead is placed in an apical position while the LV lead is placed at different positions in the coronary vein system to find the position giving the best hemodynamic performance. If applicable the AV-interval between an atrial event and the following ventricular stimulations should also be optimized before the tests to determine the merits of a lead position.

According to a further embodiment embodiment the contractility of the heart muscle is measured and the lead position or lead positions giving the highest value of the contractility is selected as the best lead position or positions. This embodiment utilizes the fact that rate of change of the impedance during contraction provides a measure of contractility. At least one of the LV or RV impedance should be measured to determine the contractility.

According to a further embodiment the ejection fraction of the heart muscle is measured and the lead position or lead positions giving the highest value of the ejection fraction is selected as the best lead position or positions. This embodiment utilizes the fact that the difference between minimum and maximum value of the impedance provides a measure of the ejection fraction.

According to still another embodiment an interval preceeding the ventricular ejection is measured and the lead position or postions giving the shortest interval is deemed to be the best lead position or postions. A segment of the impedance waveform starting with a paced QRS complex or a ventricular stimulation and terminating at a predetermined impedance value is recorded. The duration of this interval has a proportionality with the Pre-ejection time Period (PEP). Details of how the this measurement is performed and how it relates to PEP is provided in US Pat. 4,773,401. The lead position or positions providing the shortest Pre-ejection interval is deemed to be the best lead position.

According to a further embodiment of the present invention, the cardiac output is determined at each lead position and the lead position resulting in the highest cardiac output, which is the volume of blood in litres ejected by the heart per minute, is determined as the lead position resulting in the most favourable hemodynamics of the heart. Cardiac output is determined by the product of stroke volume and heart rate. It is wellknown that a measure of stoke volume can be determined through impedance measurements.

According to a further embodiment the surface ECG is recorded with the leads to be implanted att different positions. The surface ECG provides information on the synchrony between the right and left ventricles. If the lead position or lead positions are not optimal the paced QRS will have a longer duration than normal even if V-V interval between the stimulating pulses for the two ventricles is optimized. The shortest possible QRS can be achieved with the the V-V interval optimized and with the two leads in an optimal position. The lead position giving the shortest duration of the paced QRS should be searched. The morphology of the paced QRS could also be analyzed to determine the optimal lead position.
The surface ECG may be achieved from chest leads or from extremity leads.

According to still another embodiment a heart sound microphone can be placed on the patient's chest. This would allow a measurement of the patient's heart sound at different lead positions. This arrangemant would allow to identify the lead position or lead positions giving the shortest Pre-Ejection period time (PEP). The lead position giving the shortest PEP would be the most optimal lead position or lead positions hemodynamically.

According to another embodiment of the present invention, the lead position resulting in the lowest value of the quotient between PEP and left ventricular ejection time (LVET) is determined to be the lead position resulting in the most favourable hemodynamics of the heart. LVET is affected by the contractility of the myocardium and by outflow obstructions at the left ventricle. At a degraded contractility with a low stroke volume LVET will decrease, whilst it will be lengthened at outflow obstructions, such as aortic stenosis, and at a large central stroke volume. PEP tends to increase at, inter alia, cardiac insufficiency. As with PEP, LVET must be corrected with respect to the heart rate. The PEP/ LVET quotient reflects the function of the left ventricle in a more efficient way than the individual components and is not dependent on the heart rate. Hence, the quotient PEP/LVET provides an efficient and reliable measure of the heart pumping activity.

Preferably, the AV interval between stimulation of the atrium and the ventricle and/or the W interval between stimulation of the right and left ventricles are optimised before the measurement related to the determination of the performance of the actual lead position is performed.

As realized by the person skilled in the art, the method described herein, as well as preferred embodiments thereof, are suitable to realize or implement as a computer program or a computer readable medium, preferably within the contents of a device in accordance with the first aspect of the present invention and in particular within the processing means of such a device.

Further objects and advantages of the present invention will be discussed below by means of exemplifying embodiments.

### BRIEF DESCRIPTION OF THE DRAWINGS

In the following detailed description, reference will be made to the accompanying drawings, of which:
Fig. 1 shows a device in use according to the present invention for evaluating a lead position during an implantation procedure ;
Fig. 2 is block diagram of a device for evaluating the position of a medical lead under implantation through impedance measurements according to the present invention;
Fig. 3 is block diagram of a device for evaluating the position of a medical lead under implantation through ECG measurements according to the present invention;
Fig. 4 is block diagram of a device for evaluating the position of a medical lead under implantation through heart sound measurements according to the present invention;
Fig.5 shows the morphology of a surface QRS under normal conditions and with Right Bundle Branch Block (RBBB) and with Left Bundle Branch Block (LBBB).
Fig. 6 is a flow chart describing the principle steps of the process of evaluating different position of a lead of an implantable medical device to find an optimal lead site.

### DETAILED DESCRIPTION OF THE INVENTION

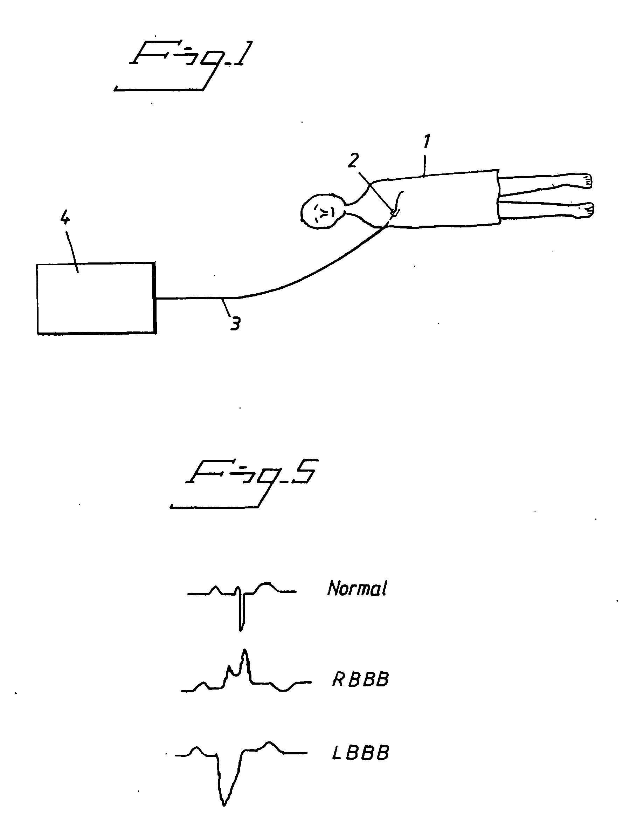

Fig. 1 illustrates the situation during the implantation during a pacemaker implantation. The patient 1 is placed on a table during the implantation procedure. The lead or leads 2 to be implanted are inserted via a suitable vein. Via this vein the leads are placed in the patient's heart under fluoroscopic supervision. Possible positions for different leads could be right atrium, the right ventricle, the left atrium or the left ventricle via the coronary sinus. Via the coronary sinus a lead can be placed in a suitable coronary vein for pacing and sensing the left ventricle of the heart. The position of the lead for pacing and sensing the left ventricle is very important for the hemodynamics. The device 4 is generally known as a Pacing System Analyzer (PSA). The device 4 is connected to the lead to be implanted via an adaptor cable 3. The device 4 is according to current practice used to measure certain important data related to the lead to be implanted. This data may be R-wave or P-wave amplitude or stimulation theshold. Further the lead impedance is frequently measured. According to the present invention the device 4 may be used to collect data on the hemodynamic performance of each lead position. The lead or leads are located at different positions and the data related to the hemodynamic performance are collected for each lead position.

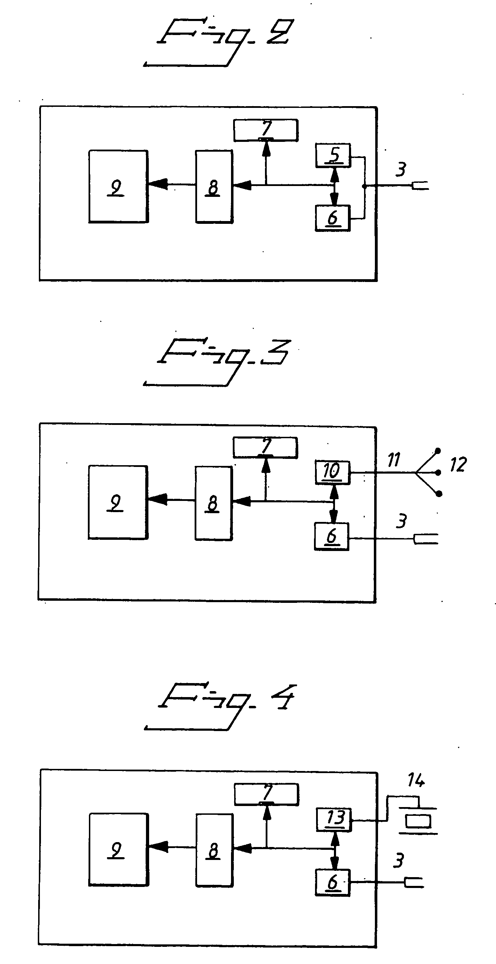

Fig. 2 shows a preferred embodiment of the present invention which utilizes sampled impedance measurements to determine the hemodynamic performance of a lead position. The pulse generating unit 6 delivers pacing pulses to one or several leads under implantation. The leads may be located in one or several of the right atrium, the left atrium, the right ventricle or the left ventricle. When the leads have been placed in a first position the impedance measuring unit 5 is activated to sample impedance values from one or several of the implanted leads. The sampling of the lead impedances is controlled by a microprocessor 8. The measured impedances varies with the blood volumes and the mechanical movements of the heart. In order to be able to follow the changes of volume or contraction pattern of a heart chamber the sampling frequency of the impedance sampling unit 5 should be 64 samples/s or more. The sampled values are stored in a memory 7. When the desired no. of lead positions have been tested and the samples have been stored in the memory 7, the samples can be analyzed through execution of an a analyzing software to determine which lead position that gives the most favourable hemodynamics. The result of the analysis may be provided to a user through the presentation unit 9.

Several different algorithms can be used to analyze the hemodynamic qualities of a given lead position. It is generally accepted that the contractility is a good measure of hemodynamics and that a high contractility is an indicator of good hemodynamic performance. In one embodiment the lead position giving the highest rate of change of the impedance is selected as the most favourable lead position. US pat 5,800,467 discloses that with greater contractility of the heart the greater volume of blood is pumped by the heart for any given heart rate. It is also disclosed that by measuring an impedance within a ventricle an indication of the contractility can be provided. The impedance can be measured as a bipolar impedance within a ventricle, preferably the right ventricle. If the impedance is measured between an epicardially located electrode on the left ventricle and an endocardially located electrode in the right ventricle an indication of left ventricular volume and left ventricular contractility can be achieved.

The algorithm to find the most favourable electrode position may also be based on detection of ejection fraction. The lead position resulting in the highest value of Ejection Fraction (EF) would be considered as the most favourable lead position. Ejection fraction can be determined by dividing stroke volume with the end-diastolic volume. US pat. 5,514,171 discloses how ejection fraction can be determined based on impedance measurements.

Another algorithm to find a favourable electrode position would be to search a position giving the shortest pre-ejection interval which is a wellknown indicator hemodynamic performance. US 4,773,401 discloses that the inteval between a paced QRS until the the right ventricular impedance crosses a zero axis in a positive direction is good measure of a pre-ejection time interval. The lead position giving the shortest pre-ejection interval is a favourable lead position.

Fig. 3 shows a preferred embodiment of the present invention which utilizes sampled surface ECG measurements to determine the hemodynamic performance of a lead position. The pulse generating unit 6 delivers pacing pulses to one or several leads under implantation. The leads may be located in one or several of the right atrium, the left atrium, the right ventricle or the left ventricle. When the leads have been placed in a first position the surface ECG measuring unit 10 is activated to sample a surface ECG. The surface ECG electrodes are placed on the patient's body or on his arms and legs. From the surface elektrogram one can determine if the atria and the ventricles are depolarized in an approriate sequence through an analysis of a sequence of the electrogram. The width or duration of a paced QRS is a wellknown indication of if the synchronization between the right and left ventricles is appropriate. After the sampled ECG has been stored in the memory 7 the microprocessor can measure the width of a paced QRS. The lead position or lead positions giving the shortest duration of the paced QRS is considered as the most favourable lead position.

In a further embodiment the morphology of the paced QRS is analyzed and the lead position which yields a surface ECG indicating the most syncronized contraction is considered as the most favourable lead position. In Fig 5 is indicated how the surface ECG morphology differs between a normal QRS and a QRS morphology indicating Right Bundle Branch Block (RBBB) and Left Bundle Branch Block (LBBB) respectively. The most favourable lead position is indicated by the QRS which from a morphology standpoint is closest to a normal QRS. The sampled surface ECG:s are stored in the memory 7. The microprocessor executes software to analyze the morphology of the stimulated QRS with different lead positions to identify the most favourable lead position. The QRS morphology analysis may be through a comparison with predetermined criteria or through a comparison with one or several templates.

Fig. 4 shows a further embodiment in which heart sounds are measured and sampled through a heart sound microphone 13 placed on a patient's body. Through wellknown analysis of heartsounds the opening and closing of the aortic valve can be detected. The Pre-Ejection Period defined as the time interval between delivery of a stimulation pulse or detection of a QRS until the opening of the aortic valve is a wellknown criteria of hemodynamic performance. The pulse generating unit 6 comprises means for detecting QRS/P-waves and for emitting stimulation pulses to the atria and to the ventricle of a patient. The heartsounds at different lead positions are measured and sampled through microphone 14, heart sound measurement unit 13 and stored in memory 7. Through an analysis of stored heart sounds by execution of a suitable software in microprocessor 8 the lead position giving the shortest PEP is identified as the most favourable lead position.

In a further improved embodiment the Left Ventricular Ejection Time is identified as the time interval between the opening and closing of the the aortic valve. The lead position giving the lowest value of the quotient PEP/LVET is consedered as a favourable lead position. The value of the quotient at different lead positions is determined through execution of a suitable software by mycroprocessor 8. The quotient PEP/LVET is a wellknown indicator of the function of the left ventricle. The normal value of the quotient PEP/LVET is 0.30-0.40 while it in situations with poor hemodynamics can be as high as 0.60.

Fig. 5 shows the morphology of a surface ECG at a normal condition and at Right Bundle Branch Block (RBBB) and at Left Bundle Branch Block (LBBB). With a proper lead position and a proper stimulation sequence QRS shape will be more similar to a normal QRS. If the stimulated QRS is sampled and stored in memory 7 for several different lead positions this information can obviously be used to select the lead position giving the most favourable conditions. Morphological critera as well as other criteria such as QRS time duration can be used.

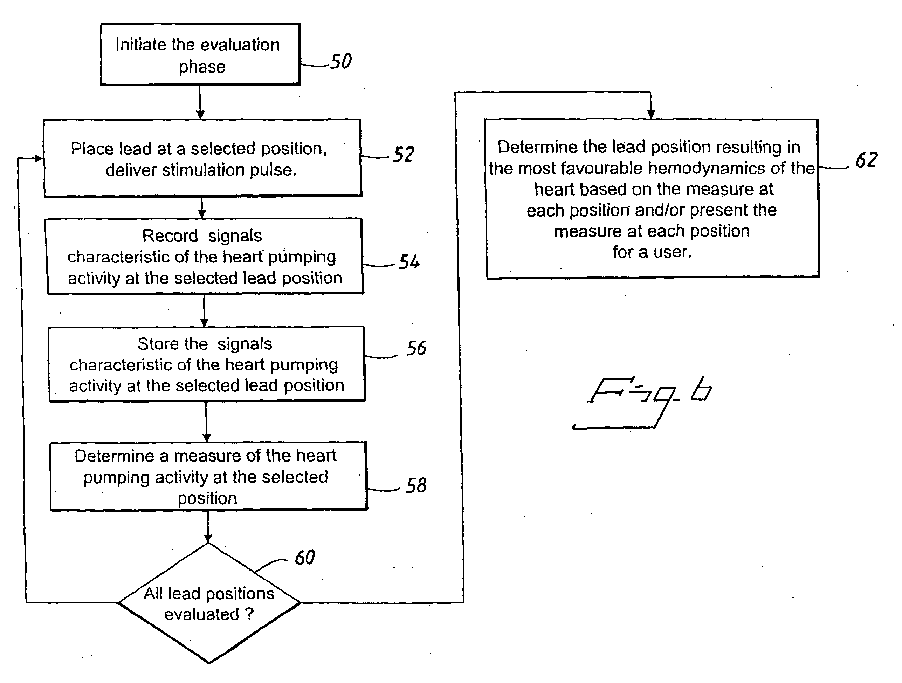

With reference now to Fig.6, a flow chart of the principles of the process of evaluating different positions of one or more leads during implantation of the medical device will be described. First, at step 50, a test phase is activated by the user. This can be performed by activating an evaluation or optimization sequence or program stored in the memory 7 of the evaluation device. As discussed above, the position or the placement of the lead is essential for the functions of the medical device as well as regards to obtaining the desired heart muscle reaction and finding or identifying the optimal lead position with respect to the heart activity is often difficult. Therefore, a number of different lead positions may have to be tested and evaluated during the implantation procedure of the medical device in order to find a placement of the lead that gives the optimal heart activity at stimulation.

Then, at step 52, the physician places the lead or leads 2 at a first position by means of a guide wire or other suitable technique, one or several stimulation pulses are delivered at the selected position using the pulse generator 6. Thereafter, at step 54, signals characteristic of the heart activity are recorded. For example, it may be intracardiac impedance signals indicating the left ventricular and/or right ventricular volume. That is, physiologic parameters reflecting hemodynamic performance are derived for each lead position. In addition, IEGM signals and/or surface ECG signals may be recorded. Also heart sounds may be recorded via a microphone 13 placed on the patient's body. Heart sounds are particularly useful to determine the opening and closing of heart valves. There are a number of different parameters that can be used as hemodynamical indicators of the heart pumping activity including the pre-ejection period (PEP), the quotient between PEP and left ventricular ejection time (LVET), the coordination between the contraction of the left ventricle and the contraction of the right ventricle, the ventricular contractility (measured as rate of change of impedance ), or the cardiac output. Subsequently, at step 56, the signals characteristic of the heart activity at the selected lead position are stored in the memory 7 of the evaluation device. As will be discussed below, the signal characteristic of the heart activity may depend on which parameter that is used in the evaluation procedure. Preferably, the AV interval between stimulation of the atrium and the ventricle and/or the W interval between stimulation of the right and left ventricles are optimised before the measurement related to the determination of the performance of the actual lead position is performed.

At step 58, the processing means determines a measure or a score value of the heart activity for the actual lead position using the recorded signal data. The measure or score value may be presented for the user on the presentation unit 9. At step 60, the user may select whether another lead position is to be evaluated or tested. If yes, the above mentioned steps 52-58 are repeated. If no, the processing means determines which lead position that results in the most favourable hemodynamics of the heart based on the determined or calculated measure for each lead position at step 62. A number of different parameters can be used for this determination. For example, the lead position resulting in the shortest pre-ejection time period (PEP) may be determined to be the optimal site with respect to the hemodynamics of the heart. The measure at each lead position together with the lead position resulting in the most favourable hemodynamics of the heart can be presented for the user visually on the presentation unit 9.

Any of the embodiments disclosed above may be used to determine the optimal lead position.

Preferably, the AV interval between stimulation of the atrium and the ventricle and/or the W interval between stimulation of the right and left ventricles are optimised before the measurement related to the determination of the performance of the actual lead position is performed in the above mentioned different embodiments of the present invention employing different approaches to derive physiologic parameters reflecting hemodynamic performance for different lead positions in order to determine the optimal lead site.

Although an exemplary embodiment of the present invention has been shown and described, it will be apparent to those having ordinary skill in the art that a number of changes, modifications, or alterations to the inventions as described herein may be made. Thus, it is to be understood that the above description of the invention and the accompanying drawings is to be regarded as a non-limiting example thereof and that the scope of protection is defined by the appended patent claims. As an example, many of the functions described above may be obtained and carried out by suitable software comprise in a micro-chip, an ASIC, or the like data carrier.

## Claims

1. Device (4) for evaluating positions of an implantable medical lead (2) connectable to said device (4) during and implantation procedure, said medical lead (2) comprising at least one electrode for stimulating and sensing, said device (4) comprising:
a pulse generating unit (6) arranged to deliver pacing pulses through said medical lead (2) at each of different positions of the lead;
measuring means (5, 6, 10, 13) arranged to sample, after delivery of said pacing pulses, one of the following:
impedance signals from said medical lead (2) at each of said different lead positions;
heart sound signals via a microphone (14) placed on a patient's body with said medical lead (2) at each of said different lead positions;
surface ECG signals with said medical lead (2) at each of said different lead positions;
intracardiac IECG signals with said medical lead (2) at each of said different lead positions;
storage means (7) arranged to store sequences of said samples, said samples being characteristic of heart pumping activity; and
processing means (8) arranged to determine , for each of said different lead positions, a respective measure, said measure being determined based on said stored sequences; said measure further reflecting the hemodynamic performance of said heart pumping activity said processing means being further arranged to determine, based on an analysis of said determined measures, the lead position of said different lead positions resulting in the most favourable hemodynamic performance of the heart with respect to said heart pumping activity according to said measures.

2. Device (4) according to claim 1, wherein said processing (8) means is arranged to determine the lead position resulting in the highest ventricular contractility, said contractility being determined from the rate of change of at least one measured impedance.

3. Device (4) according to claim 1, wherein said processing (8) means is arranged to determine the lead position resulting in the highest ventricular ejection fraction, said ejection fraction being determined from the maximum and minimum of a measured impedance.

4. Device according to claim 1, wherein said processing means (8) is arranged to determine a lead position resulting in the most synchronized contraction of the right and left chambers of the heart, said synchronized contraction being determined from a measure of at least one impedance at each lead position.

5. Device according to claim 1, wherein said processing means (8) is arranged to determine a lead position resulting in the minimisation of a pre-ejection period, said pre-ejection period being determined as the time interval starting with a paced QRS complex or a ventricular stimulation and ending when at least one ventricular impedance has reached a predetermined value, said lead position being the most favourable lead position.

6. Device according to claim 1, wherein said processing means (8) is arranged to determine a lead position resulting in the highest stroke volume, said stroke volume being determined through impedance measurements.

7. Device according to claim 1 wherein said sampled signal is a surface ECG signal and said measure determination is a morphology analysis, the lead position resulting in the stimulated QRS having the morphology closest to a normal conducted QRS being the most favourable lead position.

8. Device (4) according to claim 1 wherein said sampled signal is a surface ECG signal and said measure determination is a measurement of a paced QRS time duration, the lead position resulting in the shortest QRS duration being the most favourable lead position.

9. Device (4) according to claim 1, said device (4) comprising processing means (8) arranged to determine the lead position resulting in the most favourable hemodynamic performance of the heart based on an analysis of the heart sounds at each lead position, said analysis including detection of opening and closing of heart valves during a cardiac cycle.

10. Device according to claim 9 wherein said device (4) further comprises means (6) for measuring IECG signals and for emitting stimulating pulses to the heart via the leads (2) to be implanted at different positions, means (7) for storing said IECG signals with the leads at different positions or for storing the point in time when a stimulating pulse was delivered, processing means (8) being arranged to determine Pre-Ejection Period Time (PEP) as the interval from a paced or sensed ventricular event until the opening of an aortic valve, the most favourable electrode position being the position that gives a PEP with shortest duration.

11. Device (4) according to claim 9 wherein said device (4) further comprises means (10) for measuring surface ECG signals with the leads to be implanted at different positions, means (7) for storing said surface ECG signals with the leads (2) at different positions, said processing means (8) being arranged to determine PEP as the interval from a sensed or paced surface ECG QRS until the opening of an aortic valve, the most favourable lead position being the position that gives a PEP with shortest duration.

12. Device (4) according to claim 10 or 11 wherein said processing means is further adapted to determine LVET, said processing means being adapted to determine the quotient PEP/ LVET the most favourable lead position being determined as the lead position giving the lowest value of said quotient.

13. A device according to any preceding claim, said device being a Pacing System Analyzer.

14. Device according to any preceding claim, one lead position being in the right ventricle.

15. Device according to any one of preceding claims, connectable to at least one medical lead for placement in the right ventricle and one medical lead placed for stimulating the left ventricle.

16. Device according to claim 15, wherein the VV interval between stimulation of the right and left ventricles is optimized before the measurement related to the determination of the performance of the actual lead position is performed.

17. Device according to any one of preceding claims, connectable to at least one medical lead for placement in a ventricle, said device further being connectable to at least one lead for placement in an atrium.

18. Device according to claim 17, wherein the AV interval between stimulation of the atrium and the ventricle is optimised before the measurement related to the determination of the performance of the actual lead position is performed.

19. Device according to claim 18, wherein the AV interval between stimulation of the atrium and the ventricle and the VV interval between stimulation of the right and left ventricles are optimised before the measurement related to the determination of the performance of the actual lead position is performed.

## Patentansprüche

1. Gerät (4) zur Beurteilung von Positionen einer implantierbaren medizinischen Elektrode (2), die während eines Implantationsverfahrens an das Gerät (4) angeschlossen werden kann, wobei die medizinische Elektrode (2) mindestens eine Elektrode zur Stimulation und zum Abtasten umfasst, wobei das Gerät (4) folgende Bestandteile umfasst:
eine Pulserzeugungseinheit (6), so angeordnet, dass Schrittpulse durch die medizinische Elektrode (2) an jede der Positionen der Elektrode geleitet werden;
eine Messvorrichtung (5, 6, 10, 13), angeordnet, um nach Aussenden der Schrittpulse eine der folgenden Größen aufzunehmen:
Impedanzsignale von der medizinischen Elektrode (2) an jeder der verschiedenen Elektrodenpositionen;
Herztonsignale über ein Mikrofon (14), das auf dem Körper eines Patienten plaziert ist, mit der medizinischen Elektrode (2) an jeder der verschiedenen Elektrodenpositionen;
Oberflächen-EKG-Signale mit der medizinischen Elektrode (2) an jeder der verschiedenen Elektrodenpositionen;
Intrakardiale IEKG-Signale mit der medizinischen Elektrode (2) an jeder der verschiedenen Elektrodenpositionen;
Speichervorrichtung (7), angeordnet, um Sequenzen der Signale zu speichern, wobei die Signale charakteristisch für Herzpumpaktivität sind; und
Prozessiervorrichtung (8), angeordnet, um für jede der verschiedenen Elektrodenpositionen einen entsprechenden Messwert zu bestimmen, wobei der Messwert basierend auf den gespeicherten Sequenzen bestimmt wird; wobei der Messwert weiterhin die hämodynamische Leistung der Herzpumpaktivität wiedergibt, wobei die Prozessiervorrichtung weiterhin angeordnet ist, basierend auf der Analyse der bestimmten Messwerte, die Elektrodenposition der verschiedenen Elektrodenpositionen zu bestimmen, die die am meisten bevorzugte hämodynamische Leistung des Herzens in Bezug auf die Herzpumpaktivität gemäß den Messwerten ergibt.

2. Gerät (4) nach Anspruch 1, wobei die Prozessiervorrichtung (8) angeordnet ist, um die Elektrodenposition zu bestimmen, die die stärkste ventrikuläre Kontraktilität erzielt, wobei die Kontraktilität aus der Änderungsrate von mindestens einer gemessenen Impedanz bestimmt wird.

3. Gerät (4) nach Anspruch 1, wobei die Prozessiervorrichtung (8) angeordnet ist, um die Elektrodenposition zu bestimmen, die die höchste ventrikuläre Ejektionsfraktion erzielt, wobei die Ejektionsfraktion aus Maximum und Minimum der gemessenen Impedanz bestimmt wird.

4. Gerät nach Anspruch 1, wobei die Prozessiervorrichtung (8) angeordnet ist, um eine Elektrodenposition zu bestimmen, die in der am besten synchronisierten Kontraktion von rechter und linker Herzkammer resultiert, wobei die synchronisierte Kontraktion aus dem Messwert von mindestens einer Impedanz an jeder Elektrodenposition bestimmt wird.

5. Gerät nach Anspruch 1, wobei die Prozessiervorrichtung (8) angeordnet ist, um eine Elektrodenposition zu bestimmen, die in der Minimierung einer Präejektionsperiode resultiert, wobei die Präejektionsperiode als das Zeitintervall bestimmt wird, das mit einem getakteten QRS-Komplex oder einer ventrikulären Stimulation beginnt und mit mindestens einer ventrikulären Impedanz endet, wenn diese einen zuvor bestimmten Wert erreicht hat, wobei diese Elektrodenposition die am meisten bevorzugte Elektrodenposition ist.

6. Gerät nach Anspruch 1, wobei die Prozessiervorrichtung (8) angeordnet ist, um eine Elektrodenposition zu bestimmen, die im höchsten Schlagvolumen resultiert, wobei das Schlagvolumen durch Impedanzmessungen bestimmt wird.

7. Gerät nach Anspruch 1, wobei das abgetastete Signal ein Oberflächen-EKG-Signal ist und die Messwertbestimmung eine morphologische Analyse ist, wobei die Elektrodenposition, bei der das stimulierte QRS die Morphologie erzielt, die einem normal abgeleiteten QRS am nächsten kommt, die am meisten bevorzugte Elektrodenposition ist.

8. Gerät (4) nach Anspruch 1, wobei das abgetastete Signal ein Oberflächen-EKG-Signal ist und die Messwertbestimmung eine Messung einer getakteten QRS-Zeitspanne ist, wobei die Elektrodenposition, die die kürzeste QRS-Zeitdauer erzielt, die am meisten bevorzugte Elektrodenposition ist.

9. Gerät (4) nach Anspruch 1, wobei das Gerät (4) eine Prozessiervorrichtung (8) umfasst, die angeordnet ist, um die Elektrodenposition zu bestimmen, die, basierend auf der Analyse der Herztöne an jeder Elektrodenposition, die am meisten bevorzugte hämodynamische Leistung des Herzens erzielt, wobei die Analyse das Erkennen von Öffnen und Schließen der Herzklappen im Herzzyklus einschließt.

10. Gerät nach Anspruch 9, wobei das Gerät (4) weiterhin Folgendes umfasst:
Vorrichtung (6) zur Messung von IEKG-Signalen und zur Aussendung stimulierender Pulse an das Herz über die Elektroden (2), die an verschiedenen Positionen zu implantieren sind, Vorrichtung (7) zum Speichern der IEKG-Signale mit den Elektroden an verschiedenen Positionen oder zum Speichern des Zeitpunkts, an dem ein stimulierender Puls gesendet wurde, wobei die Prozessiervorrichtung (8) angeordnet ist, um die Zeit der Präejektionsperiode (PEP) als das Intervall von einem getakteten oder abgetasteten ventrikulären Ereignis bis zum Öffnen einer Aortenklappe zu bestimmen, wobei die am meisten bevorzugte Elektrodenposition die Position ist, die ein PEP mit der kürzesten Zeitdauer erzielt.

11. Gerät (4) nach Anspruch 9, wobei das Gerät (4) weiterhin Folgendes umfasst:
Vorrichtung (10) zur Messung des Oberflächen-EKG-Signals, wobei die Elektroden an verschiedenen Positionen zu implantieren sind, Vorrichtung (7) zum Speichern dieser Oberflächen-EKG-Signale mit den Elektroden (2) an verschiedenen Positionen, wobei die Prozessiervorrichtung (8) angeordnet wird, um das PEP als Intervall von einem abgetasteten oder getakteten Oberflächen-EKG QRS bis zum Öffnen einer Aortenklappe zu bestimmen, wobei die am meisten bevorzugte Elektrodenposition diejenige ist, die ein PEP mit der kürzesten Zeitdauer erzielt.

12. Gerät (4) nach Anspruch 10 oder 11, wobei die Prozessiervorrichtung weiter angepasst ist, um die linksventrikuläre Ejektionszeit LVET zu bestimmen, wobei die Prozessiervorrichtung weiter angepasst ist, um den Quotienten PEP/LVET zu bestimmen, wobei die am meisten bevorzugte Elektrodenposition dadurch bestimmt ist, dass die Elektrodenposition den niedrigsten Wert dieses Quotienten erzielt.

13. Gerät nach einem der vorstehenden Ansprüche, wobei das Gerät ein Schrittmachersystem-Analysator ist.

14. Gerät nach einem der vorstehenden Ansprüche, wobei eine Elektrodenposition der rechte Ventrikel ist.

15. Gerät nach einem der vorstehenden Ansprüche, das an mindestens eine medizinische Elektrode zur Plazierung im rechten Ventrikel und an eine medizinische Elektrode, plaziert zur Stimulation des linken Ventrikels, angeschlossen werden kann.

16. Gerät nach Anspruch 15, wobei das VV-Intervall zwischen Stimulation des rechten und linken Ventrikels optimiert wird, bevor die Messung in Bezug auf die Bestimmung der Leistung der aktuellen Elektrodenposition durchgeführt wird.

17. Gerät nach einen der vorstehenden Ansprüche, das an mindestens eine medizinische Elektrode zur Plazierung in einem Ventrikel angeschlossen werden kann, wobei das Gerät weiterhin an mindestens eine Elektrode zur Plazierung in einem Atrium angeschlossen werden kann.

18. Gerät nach Anspruch 17, wobei das AV-Intervall zwischen Stimulation des Atriums und des Ventrikels optimiert wird, bevor die Messung in Bezug auf die Bestimmung der Leistung der aktuellen Elektrodenposition durchgeführt wird.

19. Gerät nach Anspruch 18, wobei das AV-Intervall zwischen Stimulation des Atriums und des Ventrikels und das VV-Intervall zwischen Stimulation des rechten und linken Ventrikels optimiert werden, bevor die Messung in Bezug auf die Bestimmung der Leistung der aktuellen Elektrodenposition durchgeführt wird.

## Revendications

1. Dispositif (4) permettant d'évaluer les positions d'une dérivation médicale implantable (2) connectable audit dispositif (4) pendant une opération d'implantation, ladite dérivation médicale (2) comprenant au moins une électrode de stimulation et de détection, ledit dispositif (4) comprenant :
un module générateur d'impulsions (6) adapté pour délivrer des impulsions de stimulation par le biais de ladite dérivation médicale (2) dans chacune des différentes positions de la dérivation ;
des moyens de mesure (5, 6, 10, 13) adaptés pour échantillonner, après la délivrance desdites impulsions de stimulation, un des signaux suivants :
des signaux d'impédance de ladite dérivation médicale (2) dans chacune desdites différentes positions de la dérivation ;
des signaux de bruits cardiaques par le biais d'un microphone (14) placé sur le corps d'un patient avec ladite dérivation médicale (2) dans chacune desdites différentes positions de la dérivation ;
des signaux d'électrocardiogramme de surface avec ladite dérivation médicale (2) dans chacune desdites différentes positions de la dérivation ;
des signaux d'électrocardiogramme intracardiaque avec ladite dérivation médicale (2) dans chacune desdites différentes positions de la dérivation ;
un moyen de stockage (7) adapté pour stocker des séquences desdits échantillons, lesdits échantillons étant caractéristiques de l'action de pompage du coeur ; et
un moyen de traitement (8) adapté pour déterminer une mesure respective pour chacune desdites différentes positions de la dérivation, ladite mesure étant déterminée sur la base desdites séquences stockées, ladite mesure reflétant en outre les performances hémodynamiques de ladite action de pompage du coeur, lesdits moyens de traitement étant en outre adaptés pour déterminer, sur la base d'une analyse desdites mesures déterminées, celle desdites différentes positions de la dérivation qui donne les performances hémodynamiques du coeur les plus favorables en ce qui concerne l'action de pompage du coeur suivant lesdites mesures.

2. Dispositif (4) selon la revendication 1, dans lequel le moyen de traitement (8) est adapté pour déterminer la position de la dérivation qui donne la contractilité ventriculaire la plus élevée, ladite contractilité étant déterminée à partir de la vitesse de variation d'au moins une impédance mesurée.

3. Dispositif (4) selon la revendication 1, dans lequel le moyen de traitement (8) est adapté pour déterminer la position de la dérivation qui donne la fraction d'éjection ventriculaire la plus élevée, ladite fraction d'éjection étant déterminée à partir du maximum et du minimum d'une impédance mesurée.

4. Dispositif selon la revendication 1, dans lequel ledit moyen de traitement (8) est adapté pour déterminer une position de la dérivation qui donne la contraction la plus synchronisée des compartiments droit et gauche du coeur, ladite contraction synchronisée étant déterminée à partir d'une mesure d'au moins une impédance dans chaque position de la dérivation.

5. Dispositif selon la revendication 1, dans lequel ledit moyen de traitement (8) est adapté pour déterminer une position de la dérivation qui minimise une période de pré-éjection, ladite période de pré-éjection étant déterminée comme l'intervalle de temps qui commence avec un complexe QRS stimulé ou une stimulation ventriculaire et qui se termine lorsqu'au moins une impédance ventriculaire a atteint une valeur prédéterminée, ladite position de la dérivation étant la position la plus favorable de la dérivation.

6. Dispositif selon la revendication 1, dans lequel ledit moyen de traitement (8) est adapté pour déterminer une position de la dérivation qui donne le volume systolique le plus élevé, ledit volume systolique étant déterminé par des mesures d'impédance.

7. Dispositif selon la revendication 1, dans lequel ledit signal échantillonné est un signal d'électrocardiogramme de surface et ladite détermination de mesure est une analyse de morphologie, la position de la dérivation qui donne le QRS stimulé dont la morphologie est la plus proche de celle d'un QRS à conduction normale étant la position la plus favorable de la dérivation.

8. Dispositif (4) selon la revendication 1, dans lequel ledit signal échantillonné est un signal d'électrocardiogramme de surface et ladite détermination de mesure est une mesure d'une durée de QRS stimulé, la position de la dérivation qui donne la durée du QRS la plus courte étant la position la plus favorable de la dérivation.

9. Dispositif (4) selon la revendication 1, ledit dispositif (4) comprenant un moyen de traitement (8) adapté pour déterminer la position de la dérivation qui donne les performances hémodynamiques du coeur les plus favorables sur la base d'une analyse des bruits cardiaques dans chaque position de la dérivation, ladite analyse comprenant la détection de l'ouverture et de la fermeture de valves cardiaques pendant un cycle cardiaque.

10. Dispositif selon la revendication 9, dans lequel ledit dispositif (4) comprend en plus un moyen (6) pour mesurer des signaux d'électrocardiogramme intracardiaque et pour envoyer des impulsions de stimulation au coeur par le biais des dérivations (2) à implanter dans différentes positions, un moyen (7) pour stocker lesdits signaux d'électrocardiogramme intracardiaque avec les dérivations dans différentes positions ou pour stocker le point du temps où une impulsion de stimulation a été délivrée, le moyen de traitement (8) étant adapté pour déterminer le temps de pré-éjection (TPE) comme étant l'intervalle entre un événement ventriculaire stimulé ou détecté et l'ouverture d'une valve aortique, la position d'électrode la plus favorable étant celle qui donne la plus courte durée du TPE.

11. Dispositif (4) selon la revendication 9, dans lequel ledit dispositif (4) comprend en plus un moyen (10) pour mesurer des signaux d'électrocardiogramme de surface avec les dérivations à implanter dans différentes positions, un moyen (7) pour stocker lesdits signaux d'électrocardiogramme de surface avec les dérivations (2) dans différentes positions, ledit moyen de traitement (8) étant adapté pour déterminer le TPE comme étant l'intervalle entre un QRS détecté ou stimulé de l'électrocardiogramme de surface et l'ouverture d'une valve aortique, la position la plus favorable de la dérivation étant celle qui donne la plus courte durée du TPE.

12. Dispositif (4) selon la revendication 10 ou la revendication 11, dans lequel ledit moyen de traitement est en outre adapté pour déterminer le temps d'éjection ventriculaire gauche (TEVG), ledit moyen de traitement étant adapté pour déterminer le quotient TPE/TEVG, la position la plus favorable de la dérivation étant déterminée comme étant celle qui donne la plus faible valeur dudit quotient.

13. Dispositif selon l'une quelconque des revendications précédentes, ledit dispositif étant un analyseur de systèmes de stimulation cardiaque.

14. Dispositif selon l'une quelconque des revendications précédentes, une position de la dérivation se trouvant dans le ventricule gauche.

15. Dispositif selon l'une quelconque des revendications précédentes, connectable à au moins une dérivation médicale destinée à être placée dans le ventricule droit et à une dérivation médicale placée de manière à stimuler le ventricule gauche.

16. Dispositif selon la revendication 15, dans lequel l'intervalle VV entre la stimulation des ventricules droit et gauche est optimisé avant d'effectuer la mesure relative à la détermination des performances de la position réelle de la dérivation.

17. Dispositif selon l'une quelconque des revendications précédentes, connectable à au moins une dérivation médicale destinée à être placée dans un ventricule, ledit dispositif pouvant en outre être connecté à au moins une dérivation destinée à être placée dans une oreillette.

18. Dispositif selon la revendication 17, dans lequel l'intervalle AV entre la stimulation de l'oreillette et celle du ventricule est optimisé avant d'effectuer la mesure relative à la détermination des performances de la position réelle de la dérivation.

19. Dispositif selon la revendication 18, dans lequel l'intervalle AV entre la stimulation de l'oreillette et celle du ventricule et l'intervalle VV entre la stimulation des ventricules droit et gauche sont optimisés avant d'effectuer la mesure relative à la détermination des performances de la position réelle de la dérivation.
